# EUROPÄISCHE PATENTSCHRIFT

(11) **EP 0 745 115 B1**
(45) Veröffentlichungstag und Bekanntmachung des Hinweises auf die Patenterteilung: **10.05.2000**
(21) Anmeldenummer: 95908911.1
(22) Anmeldetag: 07.02.1995
(51) Int. Cl.: C10L 1/18, C10L 1/22, C10M 129/95, C10M 133/52, C08F 8/00, C08F 10/00

(54) **VERWENDUNG VON CARBONSÄUREESTERN ALS KRAFTSTOFF- ODER SCHMIERSTOFFADDITIVE UND EIN VERFAHREN ZU IHRER HERSTELLUNG**
USE OF CARBOXYLIC ACID ESTERS AS FUEL OR LUBRICANT ADDITIVES AND PROCESS FOR PREPARING THE SAME
UTILISATION D'ESTERS D'ACIDE CARBOXYLIQUE COMME ADDITIFS DE CARBURANTS OU DE LUBRIFIANTS ET LEUR PROCEDE DE PREPARATION

(30) Priorität: 15.02.1994 DE 4404742; 19.09.1994 DE 4433271; 16.12.1994 DE 4444912
(43) Veröffentlichungstag der Anmeldung: 04.12.1996
(73) Patentinhaber: BASF AKTIENGESELLSCHAFT, 67056 Ludwigshafen (DE)
(72) Erfinder: HÖHN, Arthur, D-67281 Kirchheim (DE); FUNKE, Hans, Boulder, CO 80304 (US); OPPENLÄNDER, Knut, D-67061 Ludwigshafen (DE); GÜNTHER, Wolfgang, D-67582 Mettenheim (DE); SCHWAHN, Harald, D-69168 Wiesloch (DE)
(86) Internationale Anmeldenummer: EP9500429
(87) Internationale Veröffentlichungsnummer: WO9521904

(56) Entgegenhaltungen:
- EP-A- 0 010 807
- EP-A- 0 148 592
- WO-A-94/13709
- DE-A- 1 912 517
- DE-A- 3 308 882

## Beschreibung

Die Erfindung betrifft die Verwendung von Carbonsäureestern der Formel I,

R¹-CO-O-R² I,

worin
- R¹: einen aliphatischen, geradkettigen oder verzweigten, Alkyl-seitenketten aufweisenden Kohlenwasserstoffrest und
- R²: einen geradkettigen oder verzweigten Kohlenwasserstoffrest mit 1 bis 30 Kohlenstoffatomen oder einen Rest der Formel II, 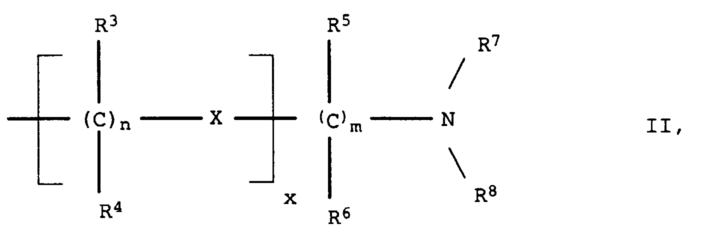 wobei die Reste
R³, R⁴, R⁵ und R⁶ unabhängig voneinander Wasserstoff, einen verzweigten oder unverzweigten Alkylrest, einen aromatischen Rest oder einen araliphatischen Rest, der auch Heteroatome enthalten kann,
R⁷ und R⁸ unabhängig voneinander verzweigte oder unverzweigte Alkylreste, aromatische oder araliphatische Reste, die auch Heteroatome enthalten können,
- X: O,S, NR⁹, PR⁹, wobei R⁹ ein aliphatischer oder aromatischer Rest ist, von denen O bevorzugt ist,
- n: und m unabhängig voneinander die Werte 2 bis 20, vorzugsweise 2 und
- X: 0 bis 30, vorzugsweise 0, bedeuten,
welche durch Umsetzung von Polypropylenen oder Polyisobutenen mit einer Molmasse von 450 bis 3000 und mindestens einer Kohlenstoff-Kohlenstoff-Doppelbindung mit Kohlenmonoxid und mit Alkoholen der allgemeinen Formel R²-OH hergestellt worden sind, als Kraft- oder Schmierstoffadditive sowie diese Additive enthaltende Kraft- oder Schmierstoffzusammensetzungen.

Vergaser und Einlaßsysteme von Ottomotoren, aber auch Einspritzsysteme für die Kraftstoffdosierung in Otto- und Dieselmotoren werden in zunehmendem Maße durch Verunreinigungen belastet, die durch Staubteilchen aus der Luft, unverbrannte Kohlenwasserstoffreste aus dem Brennraum und die in den Vergaser geleiteten Kurbelwellengehäuseentlüftungsgase verursacht werden.

Diese Rückstände verschieben das Luft-Kraftstoffverhältnis im Leerlauf und im unteren Teillastbereich, so daß das Gemisch fetter, die Verbrennung unvollständiger und wiederum die Anteile unverbrannter oder teilverbrannter Kohlenwasserstoffe im Abgas größer werden und der Benzinverbrauch steigt.

Es ist bekannt, daß zur Vermeidung dieser Nachteile Kraftstoff-additive zur Reinhaltung von Ventilen und Vergasern bzw. Einspritzsystemen verwendet werden (vgl. z.B.: M. Rossenbeck in Katalysatoren, Tenside, Mineralöladditive, Hrsg. J. Falbe, U. Hasserodt, S. 223, G. Thieme Verlag, Stuttgart 1978).

Nach der Wirkungsweise aber auch nach dem bevorzugten Wirkort solcher Detergent-Additive unterscheidet man heute zwei Generationen.

Die erste Additiv-Generation konnte nur die Bildung von Ablagerungen im Ansaugsystem verhindern, nicht aber bereits vorhandene Ablagerungen wieder entfernen, wohingegen die modernen Additive der zweiten Generation beides bewirken können (keep-clean- und clean-up-Effekt), und zwar insbesondere auch aufgrund ihrer hervorragenden Thermostabilität an Zonen höherer Temperatur, nämlich an den Einlaßventilen.

Das molekulare Bauprinzip dieser als Detergentien wirkenden Additive kann verallgemeinernd angegeben werden als Verknüpfung polarer Strukturen mit meist höhermolekularen, unpolaren oder oleophilen Resten.

Hierbei sind vor allem solche Verbindungen von Interesse, die über chlorfreie Synthesewege erhalten werden, da die Verwendung von Chlor zur Folge hat, daß chlor- oder chloridhaltige Produkte auftreten, was heute keinesfalls mehr erwünscht ist.

Derartige Detergentien, die einer Vielzahl chemischer Substanzklassen entstammen können, gelangen im allgemeinen in Kombination mit einem Trägeröl zur Anwendung. Die Trägeröle üben eine zusätzliche "Waschfunktion" aus, unterstützen und fördern oft die Detergentien in ihrer Wirkung und können zur Reduzierung der benötigten Menge an Detergens beitragen∼ Als Trägeröle werden üblicherweise viskose, hochsiedende und insbesondere thermostabile Flüssigkeiten verwendet. Sie überziehen die heiße Metalloberfläche (z.B. die Einlaßventile) mit einem dünnen Flüssigkeitsfilm und verhindern bzw. verzögern dadurch die Bildung bzw. Ablagerung von Zersetzungsprodukten an den Metalloberflächen. Geeignete Trägeröle sind zum Beispiel hochsiedende, raffinierte Mineralölfraktionen, aber auch synthetische Flüssigkeiten, wie z.B. öllösliche Addukte von Alkylenoxiden an Alkohole.

Aufgrund der oft nur geringen Detergenswirkung der als Trägerflüssigkeiten verwendeten Verbindungen und einem dementsprechend geringen Beitrag zur Gesamtperformance eines Additivpaketes hinsichtlich Detergency, ist die Einsparung an benötigten Detergentien nicht optimal und die Trägeröle selbst müssen in relativ hoher Dosierung eingesetzt werden. Üblicherweise können solche Trägeröle die Verwendung von Detergentien keinesfalls entbehrlich machen.

Aufgabe der Erfindung war es, ein Kraft- oder Schmierstoffadditiv bereitzustellen, das als Trägeröl verwendbar ist und ausgeprägte Detergenswirkung zeigt.

Die EP-A-148 592 betrifft die Herstellung von Carbonsäureestern aus Polymeren, welche eine Kohlenstoff- Kohlenstoff-Doppelbindung tragen, mit Kohlenmonoxid und einem Alkohol in Gegenwart einer Protonensäure und eines Katalysators, der mindestens eines der Metalle Palladium, Rhodium, Ruthenium, Iridium und Cobalt sowie Kupfer enthält. Der Nachweis der Produkte erfolgte durch IR-Spektroskopie. Umsatz oder Selektivität dieser Reaktion läßt sich aus den Angaben der Druckschrift nicht entnehmen. Ein Nachstellen der beschriebenen Versuche ergab, daß der Umsatz zu den gewünschten Produkten weniger als 10 % beträgt. Eine solche Ausbeute ist wirtschaftlich jedoch nicht akzeptabel.

Die DE-A 29 12 489 beschreibt die Herstellung von Carbonsäureestern durch Hydroformylierung innenständiger Olefine, die maximal 20 Kohlenstoffatome tragen, in Gegenwart eines Kobaltkatalysators unter Drücken von 150 bis 300 bar.

In der WO-A 94/13709 werden Carbonsäureester auf Basis von Poly-n-butenen, Ethylen-Propylen-Copolymeren, Ethylen-Buten-Copolymeren oder Propylen-Butylen-Copolymeren beschrieben, die Verwendung als Kraft- oder Schmierstoffadditive finden.

Es bestand daher weiterhin die Aufgabe, ein Verfahren für die Herstellung von Carbonsäuren und Carbonsäureestern aus Polymeren, welche noch eine Kohlenstoff- Kohlenstoff-Doppelbindung tragen, bereitzustellen. Ein solches Verfahren soll es ermöglichen, die gewünschten Verfahrensprodukte bei gutem Umsatz in hoher Selektivität zu gewinnen.

Demgemäß wurde die eingangs definierte Verwendung von Carbonsäureestern der Formel I gefunden.

Gegenstand der Erfindung sind außerdem Kraftstoff zusammensetzungen, die Carbonsäureester der Formel I in einer Menge von 10 bis 5000 ppm, insbesondere 100 bis 2000 ppm, enthalten.

Gegenstand der Erfindung sind außerdem Schmierstoffzusammensetzungen, welche Carbonsäureester der Formel I, in einer Menge von 0,5 bis 15 Gew.-% enthalten, insbesondere 0,5 bis 10 Gew.-%.

Die als Ausgangsmaterial zu verwendenden Polymeren sind Polypropene und Polyisobutene, die Molmassen von 450 bis 3000, vorzugsweise 700 bis 1200 haben. Solche Verbindungen sind z.B. nach der EP-A-481 297 erhältlich. Propenoligomere können z.B. nach Chem. Lett. 1525 (1991) erhalten werden.

Als Katalysatoren im genannten Verfahren dienen Metalle oder Metallverbindungen der Gruppen 8 bis 10 des Periodensystems. Diese können als Heterogen- oder bevorzugt als Homogenkatalysatoren eingesetzt werden. Als Metallverbindungen kommen z.B. die Chloride, Acetate und Nitrate der genannten Metalle in Betracht, die Metalle können aber auch elementar eingesetzt werden. Bevorzugt werden Cobalt, Palladium und Rhodium sowie deren Verbindungen. Besonders bevorzugt sind Cobaltcarbonylverbindungen sowie Cobaltsalze. Kohlenmonoxid wird in der Reaktion im Überschuß eingesetzt. Dabei wird das Verhältnis von Kohlenmonoxid zum Polymeren über den Partialdruck des Kohlenmonoxids eingestellt.

Das Polymer wird mit Kohlenmonoxid sowie mit einem Alkohol umgesetzt. Bei diesen Alkoholen handelt es sich in der Regel um primäre oder sekundäre aliphatische C₁- bis C₂₀-Alkohole wie Methanol, Ethanol, Isopropanol, n-Propanol, n-Butanol und 2-Ethyl-hexanol, weiterhin um C₂-C₃₀-Aminoalkohole wie 2-Dimethylaminoethanol, 2-Diethylaminoethanol, 2-Di-iso-propylaminoethanol, 3-Dimethylamino-1-propanol, 3-Diethylamino-1-propanol, 3-Di-iso-propylamino-1-propanol, 1-Dimethylamino-2-propanol, 1-Diethylamino-2-propanol, 1-Di-iso-propylamino-2-propanol, 3 -Dimethylamino-1,2-propandiol, um araliphatische C₇-C₁₂-Alkohole wie Benzylalkohol, um aromatische C₆-C₁₀-Alkohole wie Phenol und um C₂-C₂₀-Mono- und Polyalkylenoxidglykole und -monoether wie Ethylenglykol, Ethylenglykolmonomethylether, Diethylenglykol, Diethylenglykolmonomethylether, Triethylenglykol, Triethylenglykolmonomethylether, Tetraethylenglykol und Tetraethylenglykolmonomethylether. Im allgemeinen werden 1 bis 30 mol Alkohol, vorzugsweise 10 bis 25 mol pro Mol Polymerverbindung eingesetzt. Eine Umsetzung mit Alkoholen führt zu den entsprechenden Estern.

Vorzugsweise wird das erfindungsgemäße Verfahren in Gegenwart stickstoffhaltiger Basen durchgeführt. Als besonders geeignet haben sich dabei tertiäre aromatische Amine wie Pyridin oder Picoline herausgestellt. Diese Basen können im molaren Verhältnis von 1:1 bis 50:1, vorzugsweise 2:1 bis 15:1, bezogen auf das katalytisch aktive Metall, verwendet werden.

In der Regel beträgt das molare Verhältnis des Polymeren zum katalytisch aktiven Metall 2:1 bis 50:1, vorzugsweise 4:1 bis 15:1.

Die Reaktion kann in einem Lösungsmittel ausgeführt werden. Dabei handelt es sich im allgemeinen um polare Lösungsmittel wie Aceton, Ether wie Tetrahydrofuran, aber auch um die in der Reaktion als Reaktionspartner auftretenden Alkohole oder Wasser. Diese Lösungmittel können auch im Gemisch mit Kohlenwasserstoffen wie Alkanen verwendet werden. Die Menge des Lösungsmittels liegt im allgemeinen bei 10 bis 90 Gew.-%, bezogen auf den Gesamtansatz.

Die Reaktion kann bei Temperaturen von 20 bis 300°C, vorzugsweise bei 100 bis 200°C durchgeführt werden. Der Reaktionsdruck beträgt 50 bis 600 bar, bevorzugt ist der Bereich von 100 bis 300 bar absoluten Drucks. Die Reaktionszeiten betragen im allgemeinen 5 bis 48 Stunden. Zur Durchführung der Reaktionen werden die Reaktionspartner vermischt und anschließend unter CO-Druck auf die Reaktionstemperatur gebracht. Die Aufarbeitung kann in an sich bekannter Weise durch Abtrennung des Katalysators, Abdestillieren des Lösungsmittels sowie chromatographische Reinigung des Reaktionsproduktes erfolgen.

Die Reaktion kann kontinuierlich oder diskontinuierlich ausgeführt werden.

Das erfindungsgemäße Verfahren erlaubt die Herstellung der Verfahrensprodukte mit hoher Selektivität der Addition des Kohlenmonoxids und des Alkohols bei gutem Umsatz.

Die erfindungsgemäße Verwendung von Estern der Formel I als Kraftstoffadditive, insbesondere für Ottomotoren, erlaubt die Kombination mit bekannten Detergentien, z.B. Polyisobutylaminen, wie sie z.B. durch Hydroformylierung von Polyisobuten, gefolgt von reduktiver Aminierung erhältlich sind, und Dispergatoren als Trägerölkomponente, oder als alleinige Detergent-Komponente.

Bei der Verwendung als Schmierstoffadditiv können die Verbindungen der Formel I ebenfalls in Kombination mit weiteren üblichen Zusätzen wie z.B. Korrosionsinhibitoren, Verschleißschutzadditiven, Detergentien, Antioxidantien und Stockpunktverbesserern eingesetzt werden.

Als Detergent-Komponente in der Mischung mit den erfindungsgemäßen Stoffen kann prinzipiell jedes bekannte der hierfür geeigneten Produkte eingesetzt werden, wie sie z.B. bei J. Falbe, U. Hasserodt, Katalysatoren, Tenside und Mineralöladditive, G. Thieme Verlag, Stuttgart 1978, S. 221 f. oder bei K. Owen, Gasoline and Diesel Fuel Additives, John Wiley & Sons, 1989, S. 23 ff., beschrieben sind.

Vorzugsweise verwendet man N-haltige Detergentien, z.B. Verbindungen, die eine Amin- oder Amid-Gruppe enthalten. Insbesondere geeignet sind Polyisobutylamine gemäß EP-A-0 244 616, Ethylendiamintetraessigsäureamide und/oder -imide gemäß EP-A-0 188 786 oder Polyetheramine gemäß EP -A-0 244 725, wobei auf die Definitionen in diesen Literaturstellen Bezug genommen wird. Die dort beschriebenen Produkte verfügen herstellungsbedingt ebenfalls über den Vorteil, chlor- bzw. chloridfrei zu sein.

Die erfindungsgemäß verwendeten Carbonsäureester können auch mit herkömmlichen Trägerölen kombiniert werden. Insbesondere eignen sich Trägeröle auf Polyglykolbasis, z.B. entsprechende Ether und/oder Ester, wie sie in der US-A-5 004 478 oder der DE-A-38 38 918 beschrieben sind. Auch Polyoxyalkylenmonoole mit Kohlenwasserstoffendgruppen (US-A-4 877 416) oder Trägeröle, wie sie in der DE-A-41 42 241 offenbart sind, können eingesetzt werden.

Als Kraftstoffe für Ottomotoren kommen verbleites und insbesondere unverbleites Normal- und Superbenzin in Betracht. Die Benzine können auch andere Komponenten als Kohlenwasserstoffe, z.B. Alkohole wie Methanol, Ethanol, tert.-Butanol sowie Ether, z.B. Methyl-tert.-butylether enthalten. Neben den erfindungsgemäß verwendeten Additiven enthalten die Kraftstoffe in der Regel noch weitere Zusätze wie Korrosionsinhibitoren, Stabilisatoren, Antioxidantien und/oder weitere Detergentien.

Korrosionsinhibitoren sind meist Ammoniumsalze org. Carbonsäuren, die durch entsprechende Struktur der Ausgangsverbindungen zur Filmbildung neigen. Auch Amine zur Absenkung des PH-Wertes finden sich häufig in Korrosionsinhibitoren. Als Buntmetallkorrosionsschutz werden meist heterocyclische Aromaten eingesetzt.

Die erfindungsgemäß verwendeten Carbonsäureester leisten auch bei geringer Dosierung einen hohen Beitrag zur Gesamtperformance eines Additivpaketes hinsichtlich Detergency. Dadurch ist im allgemeinen die Einsparung von Detergentien möglich und im Einzelfall kann die Verwendung von weiteren zusätzlichen Detergentien sogar überflüssig sein.

### Beispiele

### Beispiel 1

### Herstellung von Polyisobutylcarbonsäuremethylester

In einem Autoklaven wurden 900 g Polyisobuten (Molmasse 1100; 0,82 mol), 19,2 g Dicobaltoktacarbonyl (56 mmol), 168 g 3-Picolin (1,8 mol), 600 g Methanol (18,8 mol) und 900 g eines C₁₀- bis C₁₃-Alkangemisches vorgelegt. Es wurde ein Druck von 50 bar CO aufgepreßt. Der Autoklav wurde auf 180°C erhitzt und der CO-Druck auf 270 bar gesteigert. Nach 24 Stunden wurde Luft eingeleitet, um den Katalysator zu entfernen, das Lösungsmittel sowie das Picolin wurden destillativ abgetrennt und es verblieben 920 g eines viskosen Produktes, das 71 % Polyisobutylcarbonsäuremethylester enthielt. Der Umsatz betrug 80 %, die Selektivität 89 %. Mit Pentan als Lösungsmittel ließ sich der Ester chromatographisch an Kieselgel vom nichtumgesetzten Edukt abtrennen.

### Motorentests

### Beispiel 2

In den Beispielen 2a und 2b wurde Polyisobutylcarbonsäuredimethylaminoethylester, der ausgehend von Polyisobuten mit einem zahlenmittleren Molgewicht von 1000 analog zu Beispiel 1 unter Verwendung von Dimethylaminoethanol anstelle von Methanol oder durch Umesterung von Polyisobutylcarbonsäuremethylester mit Dimethylaminoethanol hergestellt wurde, in Motorenversuchen als Kraftstoffadditiv getestet.

Die Motorenversuche wurden in einem Opel-Kadett 1,2-1-Motor nach CEC F/04/A/87 durchgeführt. Eingesetzter Kraftstoff: Euro-Super bleifrei.

Die Dosierung von Polyisobutylcarbonsäuredimethylaminoethylester sowie die erzielten Ergebnisse sind in der Tabelle aufgeführt.

**Tabelle**

| Beispiel | Dosierung [ppm] | Einlaßventilablagerungen [mg] *) | | | | |
|---|---|---|---|---|---|---|
| | | Ventile | 1 | 2 | 3 | 4 |
| 2a | 400 | | 15 (278) | 0 (132) | 3 (191) | 0 (180) |
| 2b | 200 | | 22 (278) | 7 (132) | 10 (191) | 1 (180) |

| | | | | | | |
|---|---|---|---|---|---|---|
| *) Werte in Klammern: Ablagerungen ohne Additivzusatz; die unterschiedlichen Werte sind durch Unterschiede beim eingesetzten Euro-Super bleifrei bedingt. | | | | | | |

### Beispiel 3

In den Beispielen 3a und 3b wurde Polyisobutylcarbonsäuremethylester, der gemäß Beispiel 1 hergestellt wurde, in Motorenversuchen als Kraftstoffadditiv getestet.

Die Motorenversuche wurden in einem Opel-Kadett 1,2-1-Motor nach CEC F/04/A/87 durchgeführt. Eingesetzter Kraftstoff: Euro-Super bleifrei.

Die Dosierung von Polyisobutylcarbonsäuremethylester sowie die erzielten Ergebnisse sind in der Tabelle aufgeführt.

**Tabelle**

| Beispiel | Dosierung [ppm] | Einlaßventilablagerungen [mg] *) | | | | |
|---|---|---|---|---|---|---|
| | | Ventile | 1 | 2 | 3 | 4 |
| 3a | 400 | | 4 (210) | 0 (150) | 6 (154) | 6 (200) |
| 3b | 200 | | 16 (278) | 0 (132) | 2 (191) | 8 (180) |

| | | | | | | |
|---|---|---|---|---|---|---|
| *) Werte in Klammern: Ablagerungen ohne Additivzusatz; die unterschiedlichen Werte sind durch Unterschiede beim eingesetzten Euro-Super bleifrei bedingt. | | | | | | |

## Patentansprüche

1. Verwendung von Carbonsäureestern der Formel I,
R¹-CO-O-R² I,
worin
R¹ einen aliphatischen, geradkettigen oder verzweigten, Alkylseitenketten aufweisenden Kohlenwasserstoffrest und
R² einen geradkettigen oder verzweigten Kohlenwasserstoffrest mit 1 bis 30 Kohlenstoffatomen oder einen Rest der Formel II, 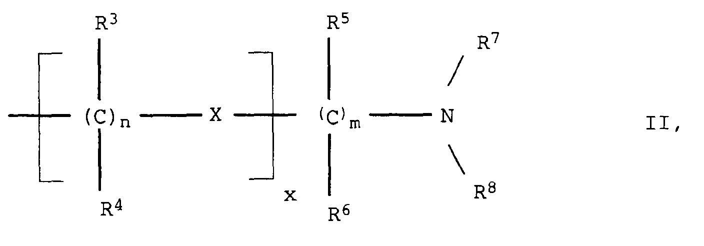
wobei die Reste
R³, R⁴, R⁵ und R⁶ unabhängig voneinander Wasserstoff, einen verzweigten oder unverzweigten Alkylrest, einen aromatischen Rest oder einen araliphatischen Rest, der auch Heteroatome enthalten kann,
R⁷ und R⁸ unabhängig voneinander verzweigte oder unverzweigte Alkylreste, aromatische oder araliphatische Reste, die auch Heteroatome enthalten können,
X O,S, NR⁹, PR⁹, wobei R⁹ ein aliphatischer oder aromatischer Rest ist,
n und m unabhängig voneinander die Werte 2 bis 20 und
x 0 bis 30 bedeuten,
welche durch Umsetzung von Polypropenen oder Polyisobutenen mit einer Molmasse von 450 bis 3000 und mindestens einer Kohlenstoff-Kohlenstoff-Doppelbindung mit Kohlenmonoxid und mit Alkoholen der allgemeinen Formel R²-OH hergestellt worden sind,
als Kraft- oder Schmierstoffadditive.

2. Verwendung von Carbonsäureestern I gemäß Anspruch 1, wobei die zur Herstellung der Carbonsäureester I eingesetzten Polypropene oder Polyisobutene eine Molmasse von 700 bis 1200 aufweisen.

3. Schmierstoffzusammensetzungen, dadurch gekennzeichnet, daß sie Carbonsäureester der Formel I gemäß den Ansprüchen 1 und 2
R¹-CO-O-R² I,
in einer Menge von 0,5 bis 15 Gew.-% enthalten.

4. Kraftstoffzusammensetzungen, dadurch gekennzeichnet, daß sie Carbonsäureester der Formel I gemäß den Ansprüchen 1 und 2
R¹-CO-O-R² I,
in einer Menge von 10 bis 5000 ppm enthalten.

## Claims

1. Use of carboxylic esters of the formula I
R¹-CO-O-R² I,
where
R¹ is an aliphatic, straight-chain or branched hydrocarbon radical having alkyl side chains and
R² is a straight-chain or branched hydrocarbon radical of 1 to 30 carbon atoms or a radical of the formula II 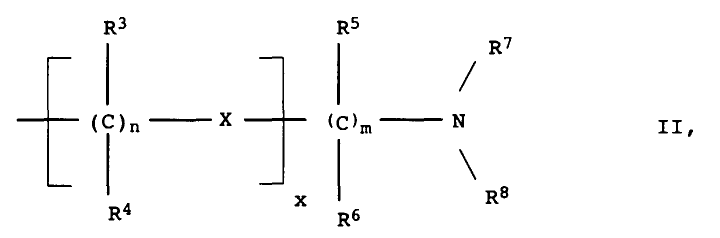 where
R³, R⁴, R⁵ and R⁶, independently of one another, are each hydrogen, branched or straight-chain alkyl, an aromatic radical or an araliphatic radical which may also contain heteroatoms,
R⁷ and R⁸, independently of one another, are each branched or straight-chain alkyl or an aromatic or araliphatic radical which may also contain heteroatoms,
X is O, S, NR⁹ or PR⁹, where R⁹ is an aliphatic or aromatic radical,
n and m, independently of one another, are each from 2 to 20, and
x is from 0 to 30,
which have been prepared by reacting polypropenes or polyisobutenes having a molar mass of from 450 to 3000 and at least one carbon-carbon double bond with carbon monoxide and with alcohols of the formula R²-OH,
as fuel additives or lubricant additives.

2. Use of carboxylic esters I as claimed in claim 1, the polypropenes or polyisobutenes used for preparing the carboxylic esters I having a molar mass of from 700 to 1200.

3. A lubricant composition which contains a carboxylic ester of the formula I as claimed in claims 1 and 2
R¹-CO-O-R² I,
in an amount of from 0.5 to 15 % by weight.

4. A fuel composition which contains a carboxylic ester of the formula I as claimed in claims 1 and 2
R¹-CO-O-R² I,
in an amount of from 10 to 5000 ppm.

## Revendications

1. Utilisation d'esters d'acide carboxylique de formule I,
R¹-CO-O-R² I,
dans laquelle
R¹ représente un reste d'hydrocarbure aliphatique, à chaîne droite ou ramifiée comprenant des chaînes alkyle latérales et
R² représente un reste d'hydrocarbure à chaîne droite ou ramifiée ayant 1 à 30 atomes de carbone ou un reste de formule II 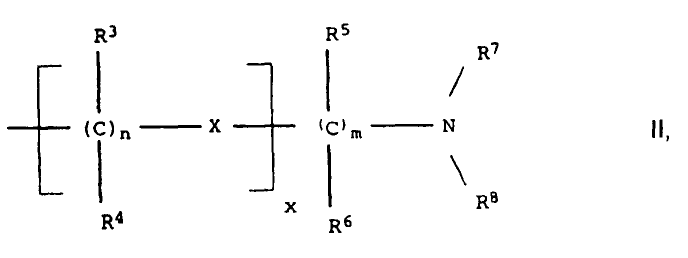 dans laquelle les restes
R³, R⁴, R⁵ et R⁶ représentent indépendamment les uns des autres un atome d'hydrogène, un reste alkyle ramifié ou non ramifié, un reste aromatique ou un reste araliphatique, qui peut également contenir des hétéroatomes,
R⁷ et R⁸ représentent indépendamment l'un de l'autre, un reste alkyle ramifié ou non ramifié, un reste aromatique ou araliphatique, qui peuvent également contenir des hétéroatomes,
X représente O, S, NR⁹, PR⁹, R⁹ étant un reste aliphatique ou aromatique,
n et m représentent indépendamment l'un de l'autre les valeurs de 2 à 20 et
x vaut de 0 à 30,
lesquels ont été produits par réaction de polypropènes ou polyisobutènes ayant une masse moléculaire de 450 à 3 000 et au moins une double liaison carbone-carbone avec le monoxyde de carbone et avec des alcools de formule générale R²-OH,
en tant qu'additifs pour carburant ou lubrifiant.

2. Utilisation d'esters d'acide carboxylique I selon la revendication 1, dans laquelle les polypropènes ou polyisobutènes mis en oeuvre pour la préparation des esters d'acide carboxylique I présentent une masse moléculaire de 700 à 1 200.

3. Compositions de lubrifiant, caractérisées en ce qu'elles contiennent des esters d'acide carboxylique de formule I selon les revendications 1 et 2,
R¹-CO-O-R² I,
à raison de 0,5 à 15% en poids.

4. Compositions de carburant, caractérisées en ce qu'elles contiennent des esters d'acide carboxylique de formule I selon les revendications 1 et 2,
R¹-CO-O-R² I,
à raison de 10 à 5000 ppm.
